(19) Europäisches Patentamt
European Patent Office
Office européen des brevets

(11) Veröffentlichungsnummer: **0 034 320**
**B1**

(12) # EUROPÄISCHE PATENTSCHRIFT

(45) Veröffentlichungstag der Patentschrift: **16.05.84**

(51) Int. Cl.³: **G 01 N 33/86,** C 12 Q 1/56

(21) Anmeldenummer: **81100899.4**

(22) Anmeldetag: **09.02.81**

(54) **Verfahren und Reagens zur Bestimmung der biologischen Aktivität von Heparin im Plasma.**

(30) Priorität: **14.02.80 DE 3005540**

(43) Veröffentlichungstag der Anmeldung: **26.08.81 Patentblatt 81/34**

(45) Bekanntmachung des Hinweises auf die Patenterteilung: **16.05.84 Patentblatt 84/20**

(84) Benannte Vertragsstaaten: **AT BE CH DE FR GB IT LI LU NL SE**

(56) Entgegenhaltungen:
**DE - A - 2 812 943**
**FR - A - 2 317 280**

**CHEMICAL ABSTRACTS, Band 86, Nr. 5, 31. Januar 1977, Seite 164, Nr. 27402v Columbus, Ohio, U.S.A. A.N. TEIEN et al.: "Assay of heparin in plasma using a chromogenic substrate for activated factor X."**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Bartl, Knut, Dr., Am Westend 6, D-8121 Wilzhofen (DE)**
Erfinder: **Lill, Helmut, Dr., Zugspitzstrasse 24, D-8121 Wielenbach (DE)**
Erfinder: **Roeschlau, Peter, Dr., Schönegertstrasse 4, D-8124 Seeshaupt (DE)**
Erfinder: **Ziegenhorn, Joachim, Dr., Ina-Seidel-Weg 1, D-8130 Starnberg (DE)**

(74) Vertreter: **Weickmann, Heinrich, Dipl.-Ing. et al, Patentanwälte Dipl.-Ing. H. Weickmann Dipl.-Phys.Dr. K. Fincke Dipl.-Ing. F.A. Weickmann Dipl.-Chem. B. Huber Dr.-Ing. H. Liska Möhlstrasse 22, D-8000 München 86 (DE)**

**EP 0 034 320 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren und Reagens zur Bestimmung der biologischen Aktivität von Heparin im Plasma

Bekannt ist ein Verfahren und ein Reagens zur Bestimmung von Heparin im Plasma, bei welchem die biologische Aktivität des Heparins direkt bestimmt wird im Gegensatz zu den bekannten Verfahren, welche zwar die vorhandene Heparinmenge, nicht aber die tatsächliche Aktivität des Heparins zu bestimmen gestatten (DE-A-2 812 943).

Dieses bekannte Verfahren besteht darin, daß man in Plasma durch Zusatz von Thrombin oder Faktor Xa als proteolytisches Enzym und eines chromogenen Substrats des Enzyms den aus dem chromogenen Substrat freigesetzten Farbstoff mißt, wobei die Bestimmung in Abwesenheit von externem Antithrombin III durchgeführt wird.

Dieses Verfahren erhöht die Aussagekraft der Heparinbestimmung und ermöglicht insbesondere eine sinnvollere Einstellung der Heparinkonzentration bei der Heparintherapie im Falle einer Thrombosegefahr. Das Verfahren ist besonders geeignet bei normaler Heparindosierung (0,1 bis 0,8 USP Haparin/ml Normalplasma). Es können jedoch bei 37° C auch Konzentrationen bis herab zu 0,02 USP/ml noch bestimmt werden. Im Niedrigstbereich zwischen 0,1 und 0,02, insbesondere zwischen 0,08 und 0,02 USP/ml Plasma sinkt jedoch die Genauigkeit der Bestimmung erheblich ab, und unter bestimmten Umständen werden von den tatsächlichen Aktivitäten erheblich abweichende Werte gefunden. Der vorliegenden Erfindung liegt daher die Aufgabe zugrunde, das bekannte Verfahren im Niedrigstbereich der Heparinaktivität zu verbessern, insbesondere hier die Empfindlichkeit und Zuverlässigkeit so zu erhöhen, daß Ausreißer vermieden werden und die Genauigkeit der Bestimmung in diesem Bereich der Genauigkeit im Normalbereich angeglichen wird.

Überraschenderweise wurde nun gefunden, daß sich diese Aufgabe durch Zusatz von Harnstoff und bestimmten Harnstoffderivaten zum Reaktionsgemisch lösen läßt.

Das erfindungsgemäße Verfahren zur Bestimmung der biologischen Aktivität von Heparin im Plasma durch Zusatz von Thrombin oder Faktor Xa als proteolytisches Enzym und eines chromogenen Substrats des Enzyms und Messung des aus dem chromogenen Substrat freigesetzten Farbstoffs, wobei die Bestimmung in Abwesenheit von externem Antithrombin III durchgeführt wird, ist dadurch gekennzeichnet, daß außerdem wenigstens eine Verbindung der allgemeinen Formel

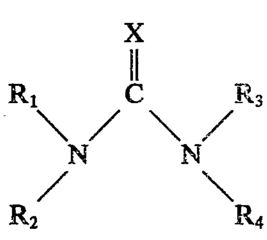

in der X ein Sauerstoffatom oder die Gruppe $NR_5$ und $R_1$, $R_2$, $R_3$, $R_4$ und $R_5$ jeweils unabhängig voneinander ein H-Atom oder eine Alkylgruppe mit 1 bis 2 C-Atomen bedeuten, in einer Menge von 0,02 bis 2,0 Mol/l, bezogen auf das Reaktionsgemisch, zugesetzt wird.

Besonders bevorzugt wird ein Zusatz zwischen 0,2 und 1,0 Mol/l, wenn X ein Sauerstoffatom ist, und von 0,05 bis 0,2 Mol/l, wenn X die Gruppe $N-R_5$ ist.

Mit dem erfindungsgemäßen Verfahren ist es möglich, selbst bei 25° C die Heparinaktivität bis herab zu ca. 0,02 USP/ml noch sehr empfindlich und genau zu bestimmten. Damit eignet sich das Verfahren der Erfindung nunmehr besonders bei der sogenannten Low-dose-Therapie zur Prophylaxe von Thrombosekomplikationen, bei welcher 0,02 bis 0,1 USP Heparin/ml verabreicht werden. Für dieses im Niedrigstbereich durchgeführte Therapieverfahren war bisher ein wirklich praktikables und automatisierbares Verfahren nicht verfügbar. Zwar wird in J. Clin. Chem. Clin. Biochem. 17 (1979) 184/85 bereits ein Verfahren zur Heparinbestimmung im angegebenen Niedrigbereich beschrieben. Dieses hat jedoch zwei wesentliche Nachteile: Zum einen wird in einer Differenzmessung ausschließlich die Wirkung des eingesetzten Heparins erfaßt. Gemäß dem Verfahren der Erfindung dagegen wird die gesamtantikoagulatorische Wirkung des Plasmas erfaßt, also der für den Arzt in der Praxis entscheidende Parameter. Außerdem wird durch die Notwendigkeit einer Differenzmessung ein zweiter Meßschritt erforderlich, wobei Plättchenfaktor IV als Heparinneutralisationsmittel zugesetzt wird. Dies behindert die Automatisierbarkeit des bekannten Verfahrens und damit auch die Anwendung in der klinischen Routine.

Für die Durchführung des Verfahrens der Erfindung gelten im übrigen die in der DE-A-2 812 943 gemachten Angaben entsprechend, mit Ausnahme des erhöhten Probeneinsatzes. So wird vorzugsweise als chromogenes Substrat ein Peptid verwendet, welches an der Carboxylgruppe eines Argininrests einen p-Nitroanilidrest durch Amidbindung gebunden aufweist. Besonders geeignet erwiesen sich im Falle der Verwendung von Thrombin als Substrat Bz-Phe-Val-Arg-pNA, H-D-Phe-Pip-Arg-pNA oder Tos-Gly-Pro-Arg-pNA. Wird als Enzym Faktor Xa verwendet, so hat sich als chromogenes Substrat Bz-Ile-Glu-Gly-Arg-pNA bewährt. Dem Reagens werden zweckmäßig außerdem Aprotinin und EDTA sowie Alkalichlorid und ein Puffer, pH 6 bis 9, außerdem gegebenenfalls auch Polyäthylenglykol, zugesetzt. Bevorzugt wird ein pH-Wert um 8 sowie Tris-Puffer (Tris-(hydroxymethyl)-aminomethan/HCl). Andere Beispiele für geeignete Puffer sind Tris/Imidazol- oder Imidazol/HCl-Puffer.

Das Verfahren kann sowohl kinetisch als auch nach der Endwertmethode durchgeführt werden. Die Auswertung erfolgt zweckmäßig über eine Eichkurve.

Ein weiterer Gegenstand der Erfindung ist ein Reagens zur Durchführung des erfindungsgemäßen Verfahrens, enthaltend

| | |
|---|---|
| 0,01 bis 0,2 Mol/l | Puffer, pH 6 bis 9, |
| 0,05 bis 0,25 Mol/l | Alkalichlorid, |
| 200 bis1100 NIH/l | Thrombin oder Faktor Xa, |
| 0,12 bis 12 mMol/l | chromogenes Substrat, |
| 0 bis 0,01 g/l | Aprotinin, |
| 0 bis 0,03 Mol/l | EDTA, |
| 0 bis 10 g/l | Polyäthylenglykol, |

welches gekennzeichnet ist durch einen Gehalt von 0,02 bis 2,0 Mol/l wenigstens einer Verbindung der allgemeinen Formel

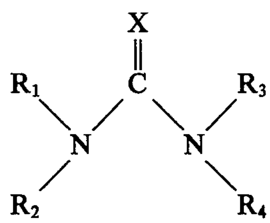

in der X ein Sauerstoffatom oder die Gruppe $NR_5$ und $R_1$, $R_2$, $R_3$, $R_4$ und $R_5$ jeweils unabhängig voneinander ein H-Atom oder eine Alkylgruppe mit 1 bis 2 C-Atomen bedeuten.

Das erfindungsgemäße Reagens ist in trockenem Zustand gut haltbar. Nach Herstellung einer gebrauchsfähigen Reagenslösung durch Zugabe von Wasser beträgt die Haltbarkeit etwa eine Woche.

Für Guanidinhydrochlorid und Harnstoff zeigt die Figur der Zeichnung die damit erzielte Empfindlichkeitssteigerung in Abhängigkeit von der zugesetzten Menge. Als Probe wurde ein Standard auf Plasma-Basis (0,1 ml) verwendet.

Die folgenden Beispiele erläutern die Erfindung weiter.

## Beispiel 1

Es wurde die Aktivität, ausgedrückt in Prozent Restaktivität, von 0,08 USP Heparin/ml Normalplasma bestimmt, wobei in einer Versuchsreihe Harnstoff zugesetzt wurde, in einer weiteren Versuchsreihe dieser Zusatz unterblieb.

2,0 ml einer Reagensmischung nachstehender Zusammensetzung:

| | |
|---|---|
| 0,2 Mol/l | Tris/HCl, pH 7,5, |
| 0,07 Mol/l | NaCl, |
| 330 NIH/l | Thrombin, |
| 0,01 g/l | Aprotinin, |
| 0,01 Mol/l | EDTA, |
| 10 g/l | Polyäthylenglykol, |

wurden mit 0,050 ml der zu untersuchenden Probe gemischt und 180 Sekunden bei 25°C inkubiert. Anschließend wurden 0,20 ml einer 1,5 mMol/l-Lösung von Tos-Gly-Pro-Arg-pNA zugesetzt und die Extinktionsdifferenz pro Minute über 2 Minuten bestimmt.

In einem weiteren Versuch wurde die Probe durch eine gleioche Menge destilliertes Wasser ersetzt. Der so erhaltene Wert wurde als Ausgangswert = 100% zugrundegelegt.

Die Bestimmung wurde in fünf Parallelversuchen durchgeführt. Die Ergebnisse sind in der Tabelle 1 angegeben.

Die Versuche wurden in gleicher Weise wiederholt, wobei jedoch erfindungsgemäß 50 g Harnstoff 0,83 Mol/l) dem Reaktionsgemisch zugesetzt wurden. Die Ergebnisse dieser Versuche sind ebenfalls in Tabelle 1 dargestellt.

Tabelle 1

Thrombinrestaktivität in Prozent

| Reagens ohne Harnstoff | Reagens mit Harnstoff (erfindungsgemäß) |
|---|---|
| 79,9 | 61,5 |
| 61,5 | 60,6 |
| 79,5 | 60,2 |
| 79,5 | 58,8 |
| 64,6 | 58,8 |

Die Versuchsergebnisse von Tabelle 1 zeigen, daß bei Durchführung des Bestimmungsverfahrens ohne Harnstoffzusatz schwankende und zum Teil stark falsche Ergebnisse auftreten. Beim erfindungsgemäßen Zusatz von Harnstoff erhält man sehr gut reproduzierbare Ergebnisse.

## Beispiele 2 bis 6

Es wurde wie in Beispiel 1 beschrieben vorgegangen, mit dem Unterschied, daß anstelle von Harnstoff die in der Tabelle 2 angegebenen Harnstoffderivate gemäß allgemeiner Formel in den in der Tabelle ebenfalls angegebenen Mengen eingesetzt wurde. Die Ergebnisse entsprachen denen von Beispiel 1.

Tabelle 2

| Beispiel | Verbindung der allgemeinen Formel | Zugesetzte Menge (Mol/l) |
|---|---|---|
| 2 | Guanidin-hydrochlorid | 0,08 |
| 3 | N-Methylharnstoff | 0,8 |
| 4 | N,N-Dimethylharnstoff | 0,4 |
| 5 | N,N'-Dimethylharnstoff | 0,8 |
| 6 | Tetramethylharnstoff | 0,2 |

**Patentansprüche**

1. Verfahren zur Bestimmung der biologischen Aktivität von Heparin im Plasma durch Zusatz von Thrombin oder Faktor Xa als proteolytisches Enzym und eines chromogenen Substrats des letzteren und Messung des aus dem chromogenen Substrat freigesetzten Farbstoffs, wobei die Bestimmung in Abwesenheit von externem Antithrombin III durchgeführt wird, dadurch gekennzeichnet, daß außerdem wenigstens eine Verbindung der allgemeinen Formel

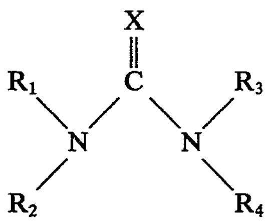

in der X ein Sauerstoffatom oder die Gruppe $NR_5$ und $R_1$, $R_2$, $R_3$, $R_4$ und $R_5$ jeweils unabhängig voneinander ein H-Atom oder eine Alkylgruppe mit 1 bis 2 C-Atomen bedeuten, in einer Menge von 0,02 bis 2,0 Mol/l, bezogen auf das Reaktionsgemisch, zugesetzt wird.

2. Verfahren nach Anspruch 1, dadurch gekennzeichnet, daß 0,2 bis 1,0 Mol/l zugesetzt werden, wenn X = O ist.

3. Verfahren nach Anspruch 1, dadurch gekennzeichnet, daß 0,05 bis 0,2 Mol/l zugesetzt werden, wenn X = $NR_5$ ist.

4. Reagens zur Durchführung des Verfahrens nach einem der vorhergehenden Ansprüche, enthaltend

| | |
|---|---|
| 0,01 bis 0,2 Mol/l | Puffer, pH 6 bis 9, |
| 0,05 bis 0,25 Mol/l | Alkalichlorid, |
| 200 bis 1100 NIH/l | Thrombin oder Faktor Xa, |
| 0,12 bis 12 mMol/l | chromogenes Substrat, |
| 0 bis 0,01 g/l | Aprotinin, |
| 0 bis 0,03 Mol/l | EDTA, |
| 0 bis 10 g/l | Polyäthylenglykol, |

gekennzeichnet durch einen Gehalt von 0,02 bis 2,0 Mol/l wenigstens einer Verbindung der allgemeinen Formel

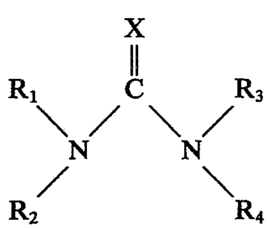

in der X ein Sauerstoffatom oder die Gruppe $NR_5$ und $R_1$, $R_2$, $R_3$, $R_4$ und $R_5$ jeweils unabhängig voneinander ein H-Atom oder eine Alkylgruppe mit 1 bis 2 C-Atomen bedeuten.

5. Reagens nach Anspruch 4, gekennzeichnet durch einen Gehalt von 0,2 bis 1,0 Mol/l einer Verbindung mit X = O.

6. Reagens nach Anspruch 4, gekennzeichnet durch einen Gehalt von 0,05 bis 0,2 Mol/l einer Verbindung mit X = $NR_5$.

**Claims**

1. Process for the determination of the biological activity of heparin in plasma by the addition of thrombin or factor Xa as proteolytic enzyme and of a chromogenic substrate of the latter and measurement of the coloures material liberated from the chromogenic substrate, whereby the determination is carried out in the absence of external antithrombin III, characterized in that there is also added at least one compound of the general formula

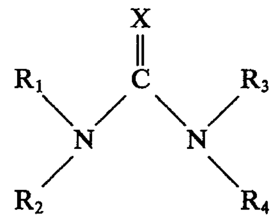

in which X signifies an oxygen atom or the group $NR_5$ and $R_1$, $R_2$, $R_3$, $R_4$ and $R_5$ each signify, independently of one another, an H atom or an alkyl group with 1 to 2 C atoms, in an amount of 0,02 to 2,0 mol/l, referred to the reaction mixture.

2. Process according to claim 1, characterized in that 0,2 to 1,0 mol/l are added when X = O.

3. Process according to claim 1, characterized in that 0,05 to 0,2 mol/l are added when X = $NR_5$.

4. Reagent for the carrying out of the process according to one of the preceding claims, containing

| | |
|---|---|
| 0,01 to 0,2 mol/l | of buffer, pH 6 to 9, |
| 0,05 to 0,25 mol/l | of alkali metal chloride, |
| 200 to 1100 NIH/l | thrombin or factor Xa, |
| 0,12 to 12 mmol/l | of chromogenic substrate, |
| 0 to 0,01 g/l | aprotonin, |
| 0 to 0,03 mol/l | EDTA, |
| 0 to 10 g/l | polyethylene glycol, |

characterized by a content of 0,02 to 2,0 mol/l of at least one compound of the general formula

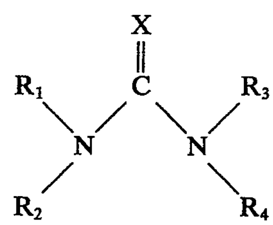

in which X signifies an oxygen atom or the group $NR_5$ and $R_1$, $R_2$, $R_3$, $R_4$ and $R_5$ each signify, independently of one another, an H atom or an alkyl group with 1 to 2 C atoms.

5. Reagent according to claim 4, characterized by a content of 0,2 to 1,0 mol/l of a compound with X = O.

6. Reagent according to claim 4, characterized by a content of 0,05 to 0,2 mol/l of a compound with X = $NR_5$.

**Revendications**

1. Procédé pour la détermination de l'activité biologique de l'héparine dans le plasma par addition de thrombine ou de facteur Xa en tant qu'enzyme protéolytique et d'un substrat chromogène de cette dernière et mesure du colorant libéré du substrat chromogène, la détermination étant effectuée en l'absence d'absence d'antithormbine III externe, caractérisé en ce que l'on ajoute en outre au moins un composé de formule générale

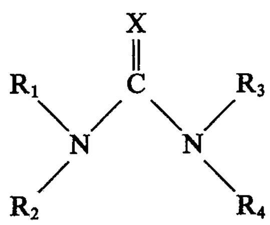

dans laquelle X représente un atome d'oxygène ou le groupe $NR_5$ et $R_1$, $R_2$, $R_3$, $R_4$ et $R_5$ représentent chacun, indépendamment les uns des autres, un atome de H ou un groupe alcoyle avec 1 à 2 atomes de C en une quantité de 0,02 à 2,0 mole/l, par rapport au mélange de réaction.

2. Procédé selon la revendication 1, caractérisé en ce que l'on ajoute de 0,2 à 1,0 mole/l lorsque X = 0.

3. Procédé selon la revendication 1, caractérisé en ce que l'on ajoute de 0,05 à 0,2 mole/l lorsque X = $NR_5$.

4. Réactif pour la mise en oeuvre du procédé selon l'une des revendications qui précèdent contenant:

| | |
|---|---|
| 0,01 à 0,2 mole/l | de tampon, pH 6 à 9, |
| 0,05 à 0,25 mole/l | de chlorure alcalin, |
| 200 à 1100 NIH/l | de thrombine ou de facteur Xa, |
| 0,12 à 12 mmoles/l | de substrat chromogène, |
| 0 à 0,01 g/l | d'aprotinine |
| 0 à 0, 03 mole/l | d'EDTA, |
| 0 à 10 g/l | de polyéthylène-glycol, |

caractérisé par une teneur de 0,02 à 2,0 moles/l d'au moins un composé de formule générale

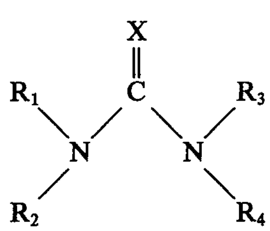

dans laquelle X représente un atome d'oxygène ou le groupe $NR_5$ et $R_1$, $R_2$, $R_3$, $R_4$ et $R_5$ représentent chacun, indépendamment les uns des autres, un atome de H ou un groupe alcoyle avec 1 à 2 atomes de C.

5. Réactif selon la revendication 4, caractérisé par une teneur de 0,2 à 1,0 mole/l d'un composé pour lequel X = O.

6. Réactif selon la revendication 4, caractérisé par une teneur de 0,05 à 0,2 mole/l d'un composé pour lequel X = $NR_5$.

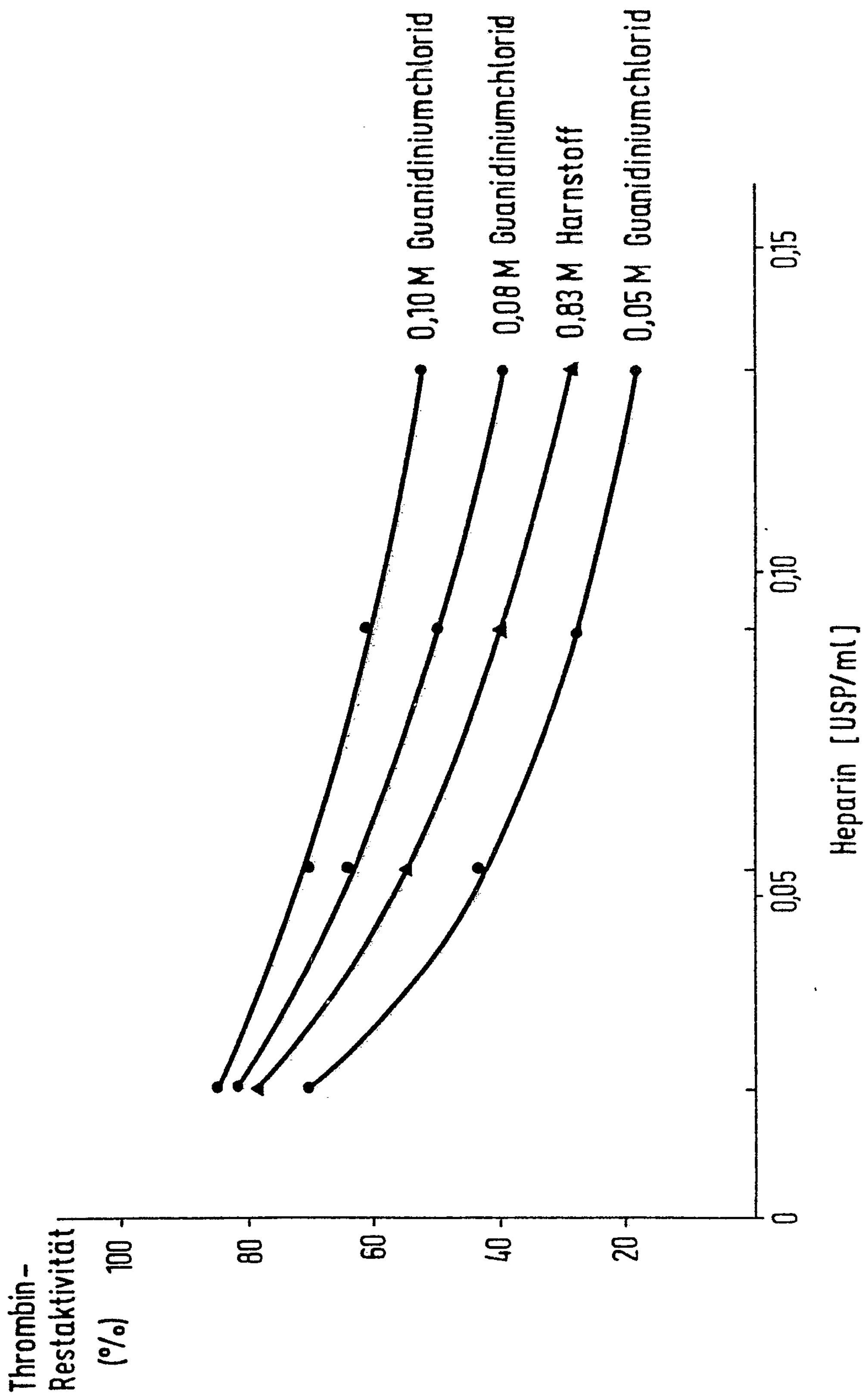
Thrombin-
Restaktivität
(%)
100
80
60
40
20
0
0,05
0,10
0,15
Heparin [USP/ml]
0,10 M Guanidiniumchlorid
0,08 M Guanidiniumchlorid
0,83 M Harnstoff
0,05 M Guanidiniumchlorid